# EUROPEAN PATENT APPLICATION

(11) **EP 4 846 933 A1**
(43) Date of publication of application: **02.12.2026**
(21) Application number: 25305754.1
(22) Date of filing: 26.05.2025
(51) Int. Cl.: A61B 5/024, A61B 5/1455, A61B 5/318, A61B 5/339, A61B 5/369, A61B 5/397, A61B 5/00, G16H 50/20, G16H 50/70

(54) **REFERENCE SIGNALS**

(71) Applicant: Koninklijke Philips N.V., 5656 AE Eindhoven (NL)
(72) Inventor: CHOFFIN, Benoît, 5656 Eindhoven (NL); KHELDOUNI, Amine, 5656 Eindhoven (NL); SHRESHTHA, Kumar, 5656 Eindhoven (NL)
(74) Representative: Philips Intellectual Property & Standards

(57) **Abstract**

Proposed concepts thus aim to provide schemes, solutions, concepts, designs, methods and systems pertaining to retrieving reference signals. In particular, embodiments aim to provide a method for retrieving reference signals by analysing the similarity / differences between an abnormality indicator in a first signal and an abnormality indicator in potential reference signals and only retrieving the reference signal if the level of similarity/difference meets at least one predetermined requirement. In other words, it is proposed that by considering the similarities/differences between indicators in the first signal and potential reference signals, the most useful reference signals for the current situation can be retrieved.

## Description

### FIELD OF THE INVENTION

This invention relates to the field of retrieving reference signals.

### BACKGROUND OF THE INVENTION

ECG is a diagnostic modality which is relatively easy to acquire signals of without expensive devices or lengthy acquisition procedures. The resulting signals can reveal much about the cardiac status of the patient, making it a beneficial entry point for cardiovascular disease (CVD) care pathways. However, interpreting an ECG signal (or other types of physiological signal) is far from trivial. It requires a deep understanding of not only the electro-physiology of the heart but also how cardiac abnormalities result in a change in the electrical signal which is recorded. In other words, there is a duality between the widespread adoption of ECGs as the main diagnostic tool for CVD and the complexity of the task of interpreting ECGs.

One way to approach this problem is to rely on diagnostic algorithms. In this way, the cardiographs in addition to outputting an ECG signal, are providing a full diagnostic analysis along with a measurements report. However, cardiographs and ECG analysis systems in general, are only meant to be used as diagnosis support for the user (e.g., a physician, a clinician, etc.). The final diagnosis lies ultimately in the hands of a human.

On the other hand, some cardiac abnormalities can be quite challenging to diagnose for a human. Not all physicians are ECG analysis experts and maintaining a high level of expertise typically requires frequent training. One of the main difficulties in diagnosing some cardiac abnormalities on an ECG stem (though this is also a difficulty when diagnosing many other types of abnormalities in other types of physiological signal) is due to the presence of abnormality mimics/lookalikes. In other words, some groups of abnormalities are similar in appearance and thus discriminating between them on a physiological signal (ECG or EEG, etc.) can prove quite challenging. This is even more important when some of these mimics might be benign while others are much more acute.

### SUMMARY OF THE INVENTION

The invention is defined by the claims.

According to examples in accordance with an aspect of the invention, there is provided a computer-implemented method for retrieving reference signals.

The method comprises: obtaining a prediction of a first abnormality indicated by a first indicator in a first physiological signal of a subject, the first physiological signal indicating a physiological parameter of the subject; and retrieving at least one reference signal, the reference signal comprising a physiological signal comprising a second indicator of a second abnormality, wherein a measure of difference between a parameter of the first indicator and a parameter of the second indicator meets a predetermined requirement.

Proposed concepts thus aim to provide schemes, solutions, concepts, designs, methods and systems pertaining to retrieving reference signals. In particular, embodiments aim to provide a method for retrieving reference signals by analysing the similarity / differences between one or more abnormality indicators in a first signal and one or more abnormality indicators in potential reference signals and only retrieving the reference signal if the level of similarity/difference meets at least one predetermined requirement.

In other words, it is proposed that by considering the similarities/differences between abnormality indicators in the first signal and potential reference signals, the most useful reference signals for the current situation can be retrieved. For instance, if a clinician is attempting to diagnose an abnormality in a first signal which includes a first abnormality indicator, a reference signal including an abnormality indicator which is similar to the first abnormality indicator (indicating the same abnormality or a different abnormality) can be retrieved such that a user is provided with an example which can aid them in making their final diagnosis.

In yet other words, the present invention provides a way to obtain reference signals which are beneficial in aiding a user's diagnosis of an abnormality in a physiological signal, to either increase confidence in their initial assessment of the abnormality being indicated in the physiological signal and/or to provide an example of another abnormality which is a better match for the abnormality indicator in the physiological signal. The inventors have realised that considering a measure of difference between parameters of abnormality indicators provides the most effective method of retrieving useful reference signals in a wide array of circumstances. For example, this can allow 'lookalike' / 'mimic' abnormalities to be found (indicators of different abnormalities which look similar), which can provide a user with plausible alternative diagnoses for the first indicator in the first physiological signal.

Ultimately, an improved method for retrieving reference signals is provided.

In some embodiments, the second abnormality may be different to the first abnormality, and the predetermined requirement for the measure of difference between the parameter of the first indicator and the parameter of the second indicator may be that the first and second indicators are sufficiently similar. This allows the retrieval of lookalike/mimic abnormalities which present themselves in similar ways (i.e., have similar indicators), and thus which a user may confuse with one another.

In some embodiments, the method may further comprise retrieving an example signal comprising a physiological signal comprising an exemplary indicator of the first abnormality. This may allow a user to be provided with an example of the first abnormality such that they can more reliably assess whether the first indicator is indeed of the first abnormality or not.

In some embodiments, the method may further comprise generating a display control signal defining the display of the at least one reference signal and the first physiological signal. This may provide an effective way to present the reference signal and the first physiological signal to a user.

In some embodiments, the display control signal may further define the display of the example signal. This may allow a user to make a more accurate assessment of which abnormality the first indicator is indicating.

In some embodiments, the display control signal may define the display of a copy of the first physiological signal alongside each of the at least one reference signals. Providing the first physiological signal next to each reference signal may provide a particularly effective display for allowing a user to compare and contrast the reference signals to the first physiological signal.

In some embodiments, the display control signal may define the display of a copy of the first physiological signal superimposed on top of each of the at least one reference signals. This may provide a particularly effective display for highlighting any deviations of the first physiological signal from the reference signals.

In some embodiments, retrieving the at least one reference signal may be further based on a measure of difference between a further parameter of the first physiological signal and a further parameter of the reference signal. This may allow more useful reference signals to be retrieved which, for example, match the first physiological signal in a further parameter (e.g., amplitude, frequency, etc.).

In some embodiments, the further parameter may be indicative of a further abnormality. This may allow more useful reference signals to be retrieved which are indicative of a further abnormality shared with the first physiological signal, thus allowing a user to see how different abnormalities may present themselves in combination with a further abnormality.

In some embodiments, the at least one reference signal may be selected from a database of physiological signals of the second abnormality based on a measure of difference between the first physiological signal and each of the physiological signals of the database of physiological signals of the second abnormality. This may allow, for example, the most similar reference signals indicating the second abnormality to be retrieved.

In some embodiments, the method may further comprise using a neural network to determine the measure of difference of each physiological signal of the database of physiological signals of the second abnormality to the first physiological signal. This may provide a particularly effective way to determine the level of similarity/difference between a plurality of physiological signals.

In some embodiments, the at least one reference signal may comprise two reference signals, and wherein the parameters of the indicators of said two reference signals respectively have a measure of difference with each other meeting a further predetermined requirement. For example, this may allow two diverse reference signals to be retrieved for the same second abnormality, thus providing a user with varied examples of how the abnormality may present itself.

In some embodiments, the prediction of the first abnormality may be input by a user. This may allow a user to influence the initial assessment of the first abnormality and thus the reference signals which are retrieved.

In some embodiments, the first physiological signal may comprise at least one of: an ECG signal; an EEG signal; an EMG signal; a PPG signal; and an SpO2 signal. The present invention may be of particular use with the above-listed physiological signals.

In some embodiments, the parameter may comprise at least one of: visual appearance; morphology; rhythm; a histogram; a Fourier transform coefficient; a wavelet transform; frequency; and amplitude. Each of these may be useful parameters to assess the measure of difference of.

According to another aspect of the invention, there is provided a computer program comprising code means for implementing the method of any herein disclosed method when said program is run on a processing system.

According to another aspect of the invention, there is provided a system for retrieving reference signals. The system comprising a processing arrangement configured to: obtain a prediction of a first abnormality indicated by a first indicator in a first physiological signal of a subject, the first physiological signal indicating a physiological parameter of the subject; and retrieve at least one reference signal, the reference signal comprising a physiological signal comprising a second indicator of a second abnormality, wherein a measure of difference between a parameter of the first indicator and a parameter of the second indicator meets a predetermined requirement.

Thus, there may be proposed concepts for retrieving reference signals, and this may be done based on a measure of difference between a parameter of an abnormality indicator in a first physiological signal and a parameter of an abnormality indicator in a potential reference signal.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for retrieving reference signals according to a proposed embodiment;
Fig. 2 is a flow diagram of a method for retrieving reference signals according to a proposed embodiment;
Fig. 3A is an example ECG presenting a STEMI pattern;
Fig. 3B is an example ECG presenting an early repolarization pattern;
Fig. 3C is an example ECG presenting a pericarditis pattern;
Fig. 4 is an example UI for presenting multiple reference signals to a user;
Fig. 5 is a flow diagram of a method for retrieving reference signals according to a proposed embodiment;
Fig. 6 is a simplified block diagram of a system for retrieving reference signals according to a proposed embodiment; and
Fig. 7 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to retrieving reference signals. According to proposed concepts, a number of possible solutions may be implemented separately or jointly. That is, although these possible solutions may be described below separately, two or more of these possible solutions may be implemented in one combination or another.

Embodiments of the invention aim to provide a method for retrieving reference signals. This can be achieved by analysing the similarity / differences between one or more abnormality indicators in a first signal and one or more abnormality indicators in potential reference signals and only retrieving the reference signal if the level of similarity/difference meets at least one predetermined requirement.

In other words, it is proposed that by considering the similarities/differences between abnormality indicators in the first signal and potential reference signals, the most useful reference signals for the current situation can be retrieved. For instance, if a clinician is attempting to diagnose an abnormality in a first signal which includes a first abnormality indicator, a reference signal including an abnormality which is similar to the first abnormality indicator (indicating the same abnormality or a different abnormality) can be retrieved such that a user is provided with an example which can aid them in making their final diagnosis.

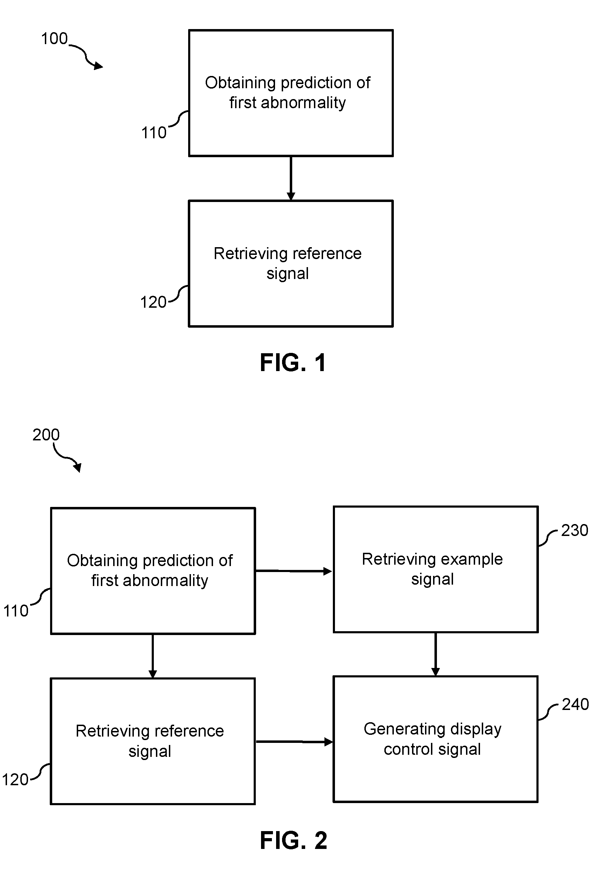

Referring now to Fig. 1, there is depicted a simplified flow diagram of a method 100 for retrieving reference signals according to a proposed embodiment.

The method 100 begins with the step 110 of obtaining a prediction of a first abnormality indicated by a first indicator in a first physiological signal of a subject, the first physiological signal indicating a physiological parameter of the subject. For example, if the first physiological signal of a subject was an EEG (indicating electrical activity in the brain of a subject), the first indicator could be spike-and-wave discharges which could be indicative of epilepsy.

In this embodiment, the first physiological signal comprises at least one of: an ECG signal; an EEG signal; an EMG signal; a PPG signal; and an SpO2 signal. The present invention is of particular use with the above-listed physiological signals, though of course, in other embodiments, the first physiological signal can comprise any suitable type of physiological signal without requiring any substantial change to the method 100, as the skilled person would understand.

The prediction of the first abnormality can be obtained by being input by a user, automatically determined by an AI/ML model or in any other suitable way.

The method 100 further comprises step 120 of retrieving at least one reference signal, the reference signal comprising a physiological signal comprising a second indicator of a second abnormality, wherein a measure of difference between a parameter of the first indicator and a parameter of the second indicator meets a predetermined requirement. A measure of difference between the same parameters of two different indicators from two different signals quantifies how much these parameters deviate from each other, and as the skilled person would appreciate, could take many different forms such as, for example, a cosine similarity. Dependent on the exact form of the measure of difference, the predetermined requirement could also take many different suitable forms, for example, a predetermined threshold which the measure of difference must be below or above. In a simple example, the measure of difference could be a percentage by which the parameters of the two indicators differ by, and the predetermined threshold could be that the measure of difference is below 10% - in this way, a reference signal including an indicator which is similar to the first indicator can be retrieved.

In this embodiment, the parameter comprises at least one of: visual appearance; morphology; rhythm; a histogram; a Fourier transform coefficient; a wavelet transform; frequency; and amplitude. Each of these are useful parameters to assess the measure of difference of, though of course, in other embodiments, the parameter can comprise any suitable type of parameter without requiring any substantial change to the method, as the skilled person would understand.

It should be noted that morphology refers to the overall shape, structure, and form of a signal. It is especially relevant for comparing waveforms, contours, or structural patterns. Useful morphological features for taking into account when considering a measure of difference between two indicators are: shape descriptors; contour matching (using methods such as Freeman Chain Code, Fourier descriptors, or Shape Context to compare shapes); Hausdorff Distance (measuring how far two contours differ); Skeletonization & Medial Axis Transform (extracting essential structure for comparison); Waveform Morphology (for time-series signals like ECG, EEG); Peak Detection & Alignment (identifying characteristic peaks (e.g., QRS complex in ECG)); Cross-Correlation of Signal Shapes (aligning and comparing waveforms directly); Dynamic Time Warping (DTW) (measuring non-linear differences in time-aligned signals); Fractal Dimension & Self-Similarity (capturing complex structures in natural signals). Rhythm refers to the periodic or quasi-periodic nature of a signal. Useful rhythmic features for taking into account when considering a measure of difference between two indicators are: Autocorrelation Function (measuring self-similarity over time to detect periodic patterns); Spectral Analysis such as Fourier / Wavelet Transform (identifying dominant frequencies and periodicity); Onset Detection & Beat Tracking (detecting transient events and rhythmic patterns).

In this embodiment, retrieving the at least one reference signal in step 120 is further based on a measure of difference between a further parameter of the first physiological signal and a further parameter of the reference signal. This allows more useful reference signals to be retrieved which, for example, match the first physiological signal in a further parameter (e.g., amplitude, frequency, etc.) and which would therefore allow for an easier direct comparison between the reference signal and the first physiological signal. In this embodiment, however, the further parameter is specifically indicative of a further abnormality (i.e., an abnormality different to the first or second abnormality). This allows more useful reference signals to be retrieved which are indicative of a further abnormality shared with the first physiological signal, thus allowing a user to see how different abnormalities may present themselves in combination with the further abnormality, therefore allowing clearer comparisons. It should be noted that the further parameter can comprise any of the types of parameters listed above (i.e., for the first and second indicators) as well as any other suitable type of parameter.

Furthermore, in this embodiment, the second abnormality is different to the first abnormality, and the predetermined requirement for the measure of difference between the parameter of the first indicator and the parameter of the second indicator is that the first and second indicators are sufficiently similar. This allows the retrieval of lookalike/mimic abnormalities which present themselves in similar ways (i.e., have similar indicators), and thus which a user may confuse with one another. In this embodiment, the reference signal can thus alternatively be referred to as a lookalike or mimic signal. It is not essential that the second and first abnormalities are different, and in other embodiments, the second abnormality can be the same as the first abnormality so that, for example, the skilled person is provided with a reference signal including a similar indicator of the same abnormality, e.g., to help confirm the prediction of the first abnormality.

In some embodiments, the at least one reference signal comprises (at least) two reference signals, and the parameters of the indicators of said two reference signals respectively have a measure of difference with each other meeting a further predetermined requirement. As described above, the measure of difference can take many different suitable forms and, in consequence, so can the further predetermined requirement. For example, the measure of difference may take the form of a percentage difference, and the further predetermined requirement may be that the percentage difference is between 40 and 80 percent, above 50 percent, or alternatively the pair of retrievable reference signals with the largest measure of difference, etc. For example, this can thus allow two suitably diverse reference signals to be retrieved for the same second abnormality, thus providing a user with varied examples of how the abnormality may present itself (with the indicator in each reference signal still remaining sufficiently similar to the first indicator). In other words, the further predetermined requirement can be that the two reference signals are sufficiently dissimilar from one another.

The at least one retrieved reference signal can then be used in a variety of ways, as would be understood by the skilled person, such as displayed to a user or output to another system, for example, an AI for further analysis, or to be combined with other data / information / signals before being displayed.

It should also be noted that the at least one retrieved reference signal can be annotated (by a human or a machine) to help aid a user in their assessment of the first indicator in the first physiological signal.

Referring now to Fig. 2, there is depicted a flow diagram of a method 200 for retrieving reference signals according to a proposed embodiment. Steps 110 and 120 are substantially the same as have been described in relation to the method 100 in Fig. 1.

In step 230, an example signal is retrieved comprising a physiological signal comprising an exemplary indicator of the first abnormality. This allows a user to be provided with an example of the first abnormality such that they can more reliably assess whether the first indicator is indeed of the first abnormality or not.

Step 240 comprises generating a display control signal defining the display of the at least one reference signal and the first physiological signal. This provides an effective way to present the reference signal and the first physiological signal to a user.

In this embodiment, the display control signal further defines the display of the example signal (retrieved in step 230) too. This allows a user to make a more accurate assessment of which abnormality the first indicator is indicating. Of course, if method 200 does not comprise step 230, then the display control signal will only define the display of the at least one reference signal and the first physiological signal.

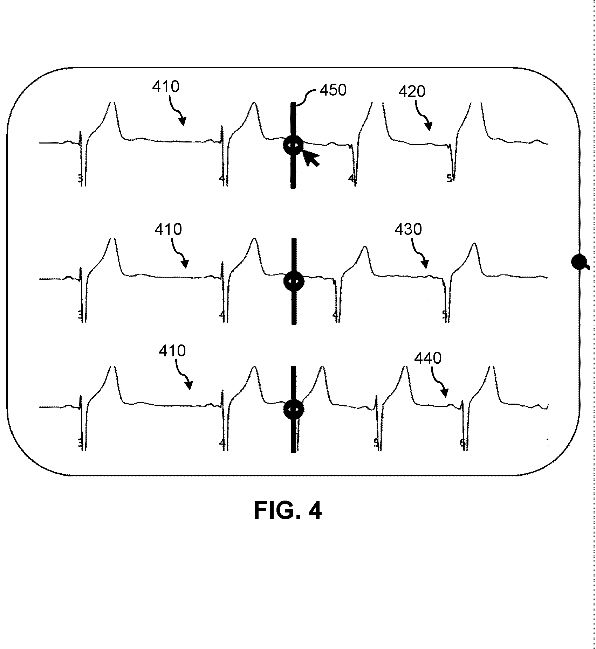

In this embodiment, the display control signal generated in step 230 defines the display of a copy/instance of the first physiological signal alongside each of the at least one reference signals. Providing the first physiological signal next to each reference signal provides a particularly effective display for allowing a user to compare and contrast the reference signals to the first physiological signal (as can be seen in Fig. 4).

In other embodiments, the display control signal can define the display of the first physiological signal and the at least one reference signal in a different manner, for example, a copy of the first physiological signal superimposed (i.e., at least partially transparently overlayed) on top of each of the at least one reference signals. This may provide another particularly effective display for highlighting any deviations of the first physiological signal from the reference signals. The signals may be superimposed such that the abnormality indicators in each signal are overlayed on top of one another, allowing more direct comparison of the morphology of each indicator.

It should be noted that if the example signal is also being displayed, a copy of the first physiological signal can also be displayed next to or superimposed on the example signal.

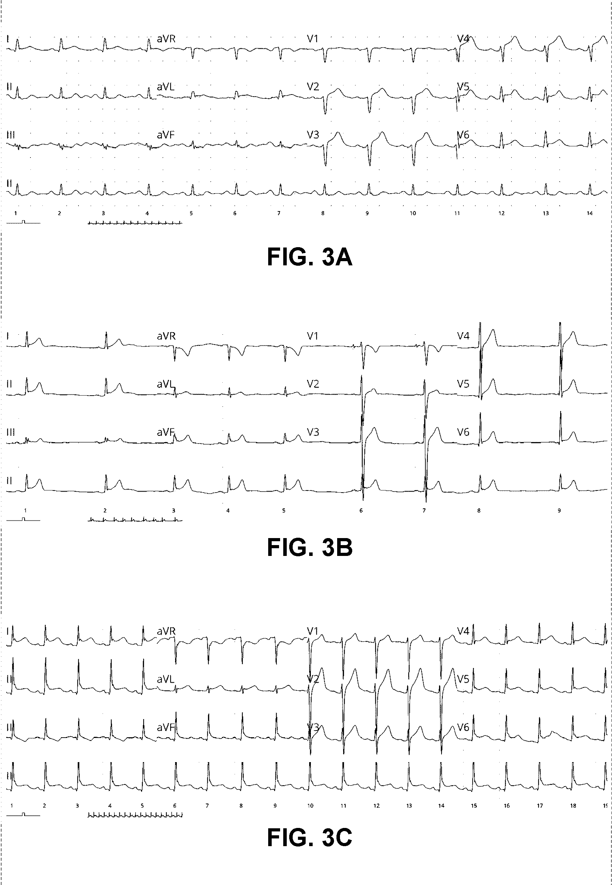

Referring now to Figs. 3A to 3C, there are depicted example ECGs presenting a STEMI pattern, an early repolarization pattern, and a pericarditis pattern respectively.

It can be seen visually that these three abnormalities have similar diagnosis criteria and therefore can easily be mistaken for one another by less skilled ECG readers. What is even more critical in this situation is that STEMI (shown in Fig. 3A) usually requires immediate reperfusion for the patient whereas early repolarization (shown in Fig. 3B) is mostly benign and does not generally require any treatment or follow-up. For ECG experts, these two abnormalities can be distinguished from the ECG combined with the clinical context of the patient (age, presence of chest pain, etc.) but for untrained professionals, distinguishing them on the ECG can be a challenge. For instance, in the emergency room or in the ambulance, the physician analysing the ECG is often not an ECG reading expert but they are the one responsible for catheterization lab activation if they suspect the presence of a STEMI. A STEMI false negative can have fatal consequences for patients, while overcalling STEMI creates an unnecessary burden for cardiologists and catheterization labs, ultimately slowing down access to care to patients who truly need it. Unnecessary invasive procedures can also have dire consequences for the 'STEMI-mimic' patient (e.g., those who actually have early repolarization or pericarditis), by generating medical complications.

Looking specifically to Fig. 3A, STEMI is diagnosed by the presence of a new ST elevation onset at the J point, of at least 1mm in all leads except V2-V3 (higher thresholds, depending on age and sex, apply here). This criterion must be present in at least two contiguous leads to raise the alarm for a STEMI.

Looking specifically to Fig. 3B, early repolarization is typically diagnosed by the presence of a non-specific, widespread and concave ST elevation in several leads of the ECG.

Looking specifically to Fig. 3C, pericarditis is diagnosed by the presence of a widespread concave ST elevation across most of the ECG leads, associated with a PR segment depression in the same leads.

Large databases pairing ECG signals and diagnoses are available and contain a wide variety of cardiac abnormalities and patient traits. Recent developments in Deep Learning can facilitate the automatic extraction of morphologically meaningful patterns from ECG (and other physiological) signals. Embeddings extracted through supervised or unsupervised learning can be used to assess the (visual / morphological) proximity of two physiological (e.g., ECG) signals. The inventors have thus realized that by combining these two advances, it becomes possible for physiological signal analysis systems to assist a user to distinguish between 'mimics' / 'lookalikes' having visually similar indicators of different abnormalities.

Referring now to Fig. 4, there is depicted an example UI for presenting multiple reference signals to a user.

The example UI comprises three sliders allowing a user to easily compare the current physiological signal 410 with each of two sampled reference signals (of the lookalike abnormalities), 430 and 440, and a sampled example signal (of the predicted abnormality), 420. The currently analyzed physiological signal 410 is on the left of each row. On the right-hand side is the sampled reference, 430 and 440, and example 420 signals. The user can, for example, move the slider 450 in each row to display more of the current or the compared physiological signal. In other embodiments of the invention, the UI could be adapted to compare each pair of signals, for instance, to superimpose beats, display more leads at once, etc.

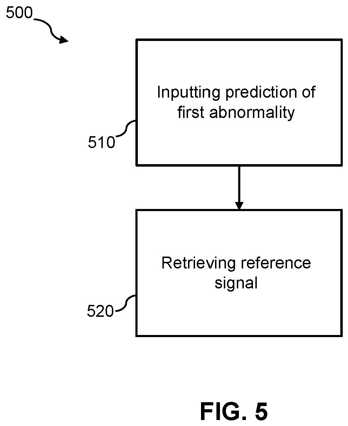

Referring now to Fig. 5, there is depicted a flow diagram of a method 500 for retrieving reference signals according to a proposed embodiment.

In step 510, the prediction of the first abnormality is obtained by being input by a user. This allows a user to influence the initial assessment of the first abnormality and thus the reference signals which are retrieved.

In step 520, the at least one reference signal is retrieved by being selected from a database of physiological signals of the second abnormality based on a measure of difference between the first physiological signal and each of the physiological signals of the database of physiological signals of the second abnormality. This allows, for example, the most similar reference signals indicating the second abnormality to be retrieved, however, this is not essential, and in other embodiments, the at least one reference signal can be retrieved by being randomly sampled from the database of physiological signals of the second abnormality (e.g., from a pool of physiological signals which all fulfil the predetermined requirement of the measure of difference). Furthermore, in this embodiment, step 520 specifically comprises using a neural network to determine the measure of difference of each physiological signal of the database of physiological signals of the second abnormality to the first physiological signal. This provides a particularly effective way to determine the level of similarity/difference between a plurality of physiological signals though is not essential, and in other embodiments, the measure of difference can be determined in any suitable way.

In summary, automated diagnosis of abnormalities in physiological signals (e.g., cardiac abnormalities on ECG machines) can be helpful for the user to help make their final diagnosis, but the accuracy of such systems is not perfect. On the other hand, reading physiological signals is a skill that is difficult to acquire and time-consuming to maintain. For instance, some cardiac abnormalities are very similar in appearance to one another on the ECG while their acuteness/seriousness may be very different.

With the present invention, a hybrid AI-human physiological signal analysis method/system is provided which will help human operators to more accurately discriminate between similar abnormalities on the physiological signal. The present method/system provides on-demand visual examples, taken from a real-world database of mimics/lookalikes and/or the predicted abnormality, to either help increase the user's confidence in their diagnosis or change it for a more accurate analysis. This thus improves clinical decision-making and increasing the speed of patient care.

In a specific example of the invention, an automated method/system is provided which allows a user to display representative beats or ECG strips from confounding alternative cardiac abnormalities. These beats or ECG strips are drawn from an annotated database of ECGs and will help the user correct their diagnosis or strengthen their confidence and their diagnosis by contrasting the currently analysed ECG to reliably annotated beats that are representative of an abnormality's mimics. Ultimately, this method/system will increase the user's trust in the ECG analysis system and reduce the number of misdiagnoses by combining human and machine analysis strength.

In a more specific first example of the invention, the proposed method/system takes as input any ECG signal (though the proposed method/system could readily be applied to any other physiological signal, as the skilled person would understand). If the user has any doubt regarding the presence of a cardiac abnormality on the ECG (whether it was diagnosed by the ECG analysis system itself or not), they have the possibility to request to be shown mimics/lookalikes (for instance, via a button, a text/spoken request, etc.). Alternatively, they could always be shown mimics/lookalikes without having to explicitly request it. Several representative beats of each of the one or more abnormality mimics/lookalikes can then be visually displayed. Taking the examples of STEMI, early repolarization and pericarditis shown in Figs. 2A-2C, all their typical morphologies are quite similar but they present a few important differences. Thus, groups of abnormalities that are all sufficiently visually similar are ideally predefined, but can be found ad hoc at runtime.

For instance, the user requests to be shown mimic/lookalike/reference signals, and the system will then fetch beats or ECG strips in a database consisting of ECG signal/diagnostics pairs (each diagnostic consisting of a set of cardiac abnormalities present on the ECG signal and reliably annotated). For each cardiac abnormality lookalike, several representative beats or ECG strips can be retrieved from the database and displayed to the user. For example, the database may be a database comprising several thousands of 10 seconds 12-lead ECGs annotated by multiple expert ECG annotators, but as the skilled person would understand, many other datasets could also be used for the present purpose.

A UI such as that shown in Fig. 3 can then be presented to the user.

For example, if the user believes that the ECG signal presents a STEMI cardiac abnormality (as shown in Fig. 2A), but they are not completely sure of their diagnosis, they can request to be shown reference (i.e., lookalike) signals and example (i.e., examples of the predicted first abnormality) signals. The system can then fetch at least one beat presenting each of STEMI, pericarditis, and early repolarization to be displayed to the user (e.g. using the UI of Fig. 3). By comparing the current ECG signal to the reference and example beats, the user can strengthen their confidence in their diagnosis by: making sure that their ECG is closest to the example ECG(s) with a reliable annotation of STEMI; or changing their diagnosis to either early repolarization or pericarditis (and ultimately changing the patient care pathway for the better), for example, if they discover that one of the reference signals is a better match for the pattern present in the current ECG.

In a specific example of the invention, there are three different proposed methods for selecting the representative beats or physiological signal strips:
1. Random sampling: the user selects an amount of X random physiological signals to sample from the database for each abnormality reference signal. To avoid visual clutter, a maximum number of reference signals can be selected by the user and/or predefined in the system.
2. Diversity sampling: This approach will select representative reference signals for each lookalike abnormality, with the objective to provide the user with diverse (in terms of abnormality patterns) reference signals for each abnormality. For example, ECG cardiac abnormalities are generally defined by clear-cut criteria but in the real-world, cardiac abnormalities are represented by a spectrum of patterns on the ECG, from the most extreme to the most subtle morphology. Some exceptions may also exist (for instance, atypical Left Bundle Branch Blocks). This approach has the advantage of giving the user a diverse sample of reference signals to give them a more complete picture of each of the abnormality mimics. It would require an embedding space to determine how close two reference signals are, and a strategy to sample diversely from each of the mimics in this space (e.g. k-Determinantal Point Processes). This strategy would be the most suited for educational purposes as it would allow the trainee to see a wide variety of reference signals for each abnormality and help them learn to discriminate between different mimics.
3. Proximity sampling: This final approach aims at selecting reference signals for each lookalike abnormality based on their morphology proximity with the currently analysed physiological signal. For example, two reference signals for each lookalike abnormality are selected: the most representative signal of the lookalike abnormality taken from the database; and the signal of the lookalike abnormality which is morphologically the closest to the physiological signal being currently analysed.
Sampling these two reference signals will give the user a sense of the underlying distribution of the signal morphologies for each of the lookalike abnormalities, biased towards the current physiological signal. In other words, the user will be shown two examples of each mimic: one that is typical and one that is most similar in morphology/shape/appearance to the current physiological signal. The idea is to give the user reasons to doubt and reasons to agree with each of the possible diagnoses, to inform their final decision as much as possible. Again, this strategy would benefit from an embedding space to determine how close two signals are or, alternatively, using a discrete set of parameters representing the morphology. This embedding can be generated by training a deep neural network in a self-supervised manner. Once this is defined, it's straightforward to select the most representative lookalike abnormality signal (e.g., take the centre of the cluster of beats/ECG strips presenting a given abnormality mimic) and the closest lookalike abnormality signal (e.g., take the one minimizing a given distance with the current physiological signal in this pre-defined embedding space). By selecting the closest lookalike abnormality signal, it may happen that the system chooses a signal from the same patient if they are present in the database. The user may have the choice to remove all physiological signals from the same patient from the sampling pool.

It should be noted that in the specific example above, the focus has been on morphology mimics. However, in other embodiments of the invention, other relevant physiological signal dimensions/parameters can be considered to define a reference signal (e.g., a lookalike abnormality): for instance, rhythm. In this example, Atrial fibrillation, Atrial flutter and Atrial tachycardia could be considered as three abnormality mimics - it would then require defining beat/ECG strip proximity based on rhythm rather than beat morphology in the latent embedding space.

In a second specific example of the invention, a database of around 10,000 ECGs can be leveraged, strongly annotated by an ECG expert and with a wide diversity of cardiac abnormalities. The embedding of each ECG signal of his database can first be computed using a pre-trained Deep Learning ECG foundation model. This embedding can capture the similarity of patterns between different ECG signals. Much like in the first specific example described above, a human user is analysing an ECG signal and trying to decide if the patient has a STEMI or not. For simplicity, we will again assume that STEMI has only two mimics/lookalikes (though in reality there are more): early repolarization (benign) and pericarditis (acute). As discussed above, STEMI is also highly acute.

The user then inputs their prediction of STEMI and requests to be shown lookalike abnormalities (i.e., reference signals), such that they will be provided with ECG examples of cardiac abnormalities presenting similar patterns as STEMI. In this embodiment, proximity sampling, as described above, is implemented. The system computes an embedding vector for the current ECG signal (or retrieves it if it has already been computed) and then, for each abnormality of the group of lookalike/mimic abnormalities as well as the predicted abnormality:
- Fetches from among all ECGs diagnosed with the mimic (or the prediction, i.e., STEMI) in the database, the closest to the centre (of the morphological/rhythm distribution): this ECG represents the 'typical' ECG with this abnormality. It can help the user remember what typical patterns are expected for diagnosing this mimic.
- Fetches from among all ECGs diagnosed with the mimic (or the prediction, i.e., STEMI) in the database, the closest to the embedding of the currently analysed ECG: this ECG represents the closest to the analysed ECG, i.e., the smallest measure of difference.

The closest and average physiological signal for each lookalike abnormality / mimic should both give the user an idea of the spectrum of patterns to expect from each mimic and give the user, by contrast, reasons to believe or doubt that their current diagnosis is the correct one. For instance, showing a very similar physiological signal but with a slight difference in morphology will help the user strengthen their confidence in their current diagnosis.

To determine the distance from one embedding vector (e.g., one ECG signal) to another one, it is paramount to define a relevant diagnosis metric. For example, the cosine similarity could be used, but as the skilled person would understand, other suitable metrics are also available.

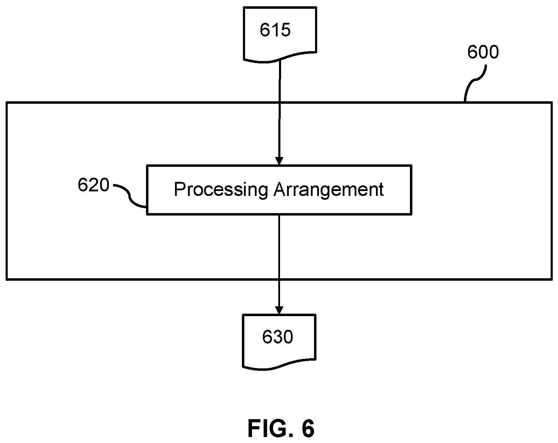

Referring now to Fig. 6, there is depicted a system 600 for retrieving reference signals according to a proposed embodiment. The system 600 comprises a processing arrangement 620.

The processing arrangement 620 can be configured to perform any of the herein-disclosed methods (e.g., method 100, 200, or 500), however, in this embodiment, the processing arrangement 620 is specifically configured to perform method 100, i.e., to: obtain a prediction of a first abnormality indicated by a first indicator in a first physiological signal of a subject, the first physiological signal indicating a physiological parameter of the subject; and retrieve at least one reference signal, the reference signal comprising a physiological signal comprising a second indicator of a second abnormality, wherein a measure of difference between a parameter of the first indicator and a parameter of the second indicator meets a predetermined requirement.

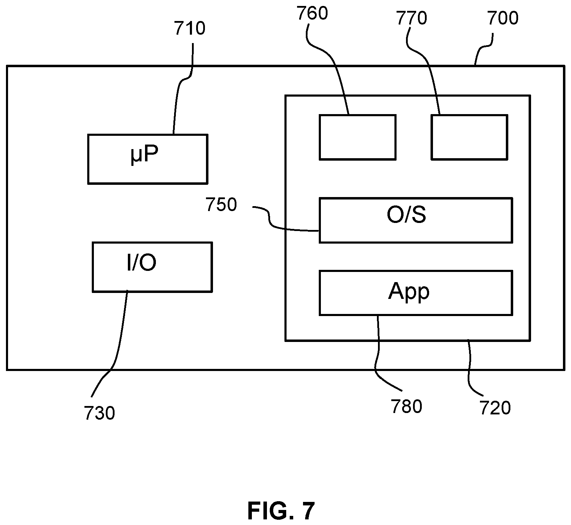

Fig. 7 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-2 and 5, and the system of Fig. 6, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for retrieving reference signals, the method comprising:
obtaining a prediction of a first abnormality (110) indicated by a first indicator in a first physiological signal of a subject, the first physiological signal indicating a physiological parameter of the subject; and
retrieving at least one reference signal (120), the reference signal comprising a physiological signal comprising a second indicator of a second abnormality, wherein a measure of difference between a parameter of the first indicator and a parameter of the second indicator meets a predetermined requirement.

2. The computer-implemented method of claim 1, wherein the second abnormality is different to the first abnormality, and the predetermined requirement for the measure of difference between the parameter of the first indicator and the parameter of the second indicator is that the first and second indicators are sufficiently similar.

3. The computer-implemented method of claim 1 or 2, further comprising retrieving an example signal (230) comprising a physiological signal comprising an exemplary indicator of the first abnormality.

4. The computer-implemented method of any prior claim, wherein the method further comprises generating a display control signal (240) defining the display of the at least one reference signal and the first physiological signal, and optionally, if dependent on claim 3, the display of the example signal.

5. The computer-implemented method of claim 4, wherein the display control signal defines the display of a copy of the first physiological signal alongside each of the at least one reference signals.

6. The computer-implemented method of claim 4, wherein the display control signal defines the display of a copy of the first physiological signal superimposed on top of each of the at least one reference signals.

7. The computer-implemented method of any prior claim, wherein retrieving the at least one reference signal is further based on a measure of difference between a further parameter of the first physiological signal and a further parameter of the reference signal, and optionally wherein the further parameter is indicative of a further abnormality.

8. The computer-implemented method of any prior claim, wherein the at least one reference signal is selected (520) from a database of physiological signals of the second abnormality based on a measure of difference between the first physiological signal and each of the physiological signals of the database of physiological signals of the second abnormality.

9. The computer-implemented method of claim 8, wherein the method further comprises using a neural network to determine the measure of difference of each physiological signal of the database of physiological signals of the second abnormality to the first physiological signal.

10. The computer-implemented method of any prior claim, wherein the at least one reference signal comprises two reference signals, and wherein the parameters of the indicators of said two reference signals respectively have a measure of difference with each other meeting a further predetermined requirement.

11. The computer-implemented method of any prior claim, wherein the prediction of the first abnormality is input by a user (510).

12. The computer-implemented method of any prior claim, wherein the first physiological signal comprises at least one of: an ECG signal; an EEG signal; an EMG signal; a PPG signal; and an SpO2 signal.

13. The computer-implemented method of any prior claim, wherein the parameter comprises at least one of: visual appearance; morphology; rhythm; a histogram; a Fourier transform coefficient; a wavelet transform; frequency; and amplitude.

14. A computer program product comprising computer program code means which, when executed on a computing device having a processing system, cause the processing system to perform all of the steps of the computer-implemented method according to any of claims 1 to 13.

15. A system (600) for retrieving reference signals, the system comprising:
a processing arrangement (620) configured to:
obtain a prediction of a first abnormality indicated by a first indicator in a first physiological signal of a subject, the first physiological signal indicating a physiological parameter of the subject; and
retrieve at least one reference signal, the reference signal comprising a physiological signal comprising a second indicator of a second abnormality, wherein a measure of difference between a parameter of the first indicator and a parameter of the second indicator meets a predetermined requirement.
